# EUROPEAN PATENT SPECIFICATION

(11) **EP 3 294 392 B1**
(45) Date of publication and mention of the grant of the patent: **30.12.2020**
(21) Application number: 16792305.1
(22) Date of filing: 09.05.2016
(51) Int. Cl.: A61M 15/00, A61M 11/00

(54) **NEBULIZERS HAVING YAWN DETECTION**
ZERSTÄUBER MIT GÄHNENERKENNUNG
NÉBULISEURS AVEC DÉTECTION DU BÂILLEMENT

(30) Priority: 10.05.2015 US 201562159315 P; 28.12.2015 US 201562271366 P
(43) Date of publication of application: 21.03.2018
(73) Proprietor: Omega Life Science Ltd, 23101 Migdal Haemek (IL)
(72) Inventor: HAZANI, Miron, 3438108 Haifa (IL)
(74) Representative: Russell, Tim
(86) International application number: PCT/IL2016/050491
(87) International publication number: WO 2016/181390

(56) References cited:
- WO-A1-2005/102428
- WO-A1-2013/008150
- WO-A2-2008/048234
- US-A- 5 277 175
- US-A1- 2004 123 863
- US-A1- 2008 082 139
- US-A1- 2008 082 139
- US-A1- 2010 031 964
- US-A1- 2010 078 015
- US-A1- 2013 228 169
- US-B1- 6 598 602
- US-B1- 6 606 989
- US-B2- 8 944 052

## Description

### TECHNICAL FIELD

The present disclosure generally relates to the field of nebulizers for aerosol generation and methods of using same for treating diseases and disorders.

### BACKGROUND

Nebulizers are commonly used for delivering aerosol medication to patients via the respiratory system. Two main goals of inhalation include promoting a more rapid onset of drug action and decreasing doses of medications. Currently there are three major categories of dispensers for lung deposition of drugs: pressurized metered-dose inhalers (PMDIs), dry powder inhalers (DPI) and nebulizers.

To limit drug waste during exhalation, breath-enhanced nebulizers, breath-actuated nebulizers (BANs), and nebulizers with an attached storage bag and a one-way mouthpiece valve have been developed. For example, the breath actuated AeroEclipse® II nebulizer, creates aerosol only during the inspiratory phase.

Conventional aerosol delivery systems and the availability of new technologies have led to the development of "intelligent" nebulizers, such as the I-neb Adaptive Aerosol Delivery (AAD) System. This system has been designed to continuously adapt to changes in the patient's breathing pattern, and to pulse aerosol only during the inspiratory part of the breathing cycle. US 8 944 052 discloses a system for aerosol delivery based on a control signal.

With regards to PMDIs, poor coordination of canister actuation and inspiration often prevents adequate metered-dose inhaler (MDI) usage by patients. Breath-actuated inhalers (BAIs) have been developed to prevent this problem. BAIs also deliver a pressurized aerosol metered dose of drug, and are automatically actuated when the user inhales through the mouthpiece.

A yawn is a reflex known to trigger a deep inhalation.

US 2008/082139 discloses a means of stimulating a yawn for the purpose of improving inhalation therapy; WO 2013/008150 concerns a system for detecting alertness level in a user, including detection of a yawn.

### SUMMARY

There is provided a system for aerosols delivery, the system comprising an aerosol delivery device comprising a controllable aerosol release mechanism configured to release aerosols based on a control signal; characterized in that the system further comprises a yawn detector configured to provide a yawn indicative signal in a subject; and a processing circuity configured to identify a yawn based on said yawn indicative signal and to provide a control signal to said aerosol release mechanism, thereby affect release of aerosols from said device, preferably wherein said processing circuitry is further configured to stimulate yawning in the subject.

In some embodiments identification of the yawn is facilitated by a facial recognition program capable of recognizing facial gestures associated with yawning, preferably wherein said facial gestures comprise a deep inhalation maneuver.

In some embodiments the system further comprises a yawn stimulator configured to stimulate the yawning.

In some embodiments the yawn stimulator is configured to provide a still image, dynamic image, sound, scent, flavor, sensation or any combination thereof.

Without wishing to be bound by any theory or mechanism, the yawn stimulating signals may induce yawning through a "contagious yawning" mechanism.

In some embodiments the yawning stimulation is activated by the subject or a caregiver.

In some embodiments the yawning stimulation is activated automatically.

In some embodiments the aerosol delivery device is an inhaler or a nebulizer.

In some embodiments the aerosol delivery device is selected from the group consisting of: a pressurized meter dose inhaler, dry particle inhaler or soft mist inhaler.

It is to be understood that a yawn includes a phase of deep inhalation. Typically, this phase occurs just before the widest opening of the mouth and closing of the eyes take place.

In some embodiments the processing circuitry is configured to predict a yawn based on the yawn indicative signal, and to provide a control signal to the aerosol release mechanism, based on said prediction, thereby schedule release of aerosols from the aerosol delivery device.

In some embodiments the aerosol release is a bolus aerosol release.

In some embodiments the aerosol comprises a pharmaceutical composition.

In some embodiments there is provided the system as described herein for use in the treatment of a pulmonary disease or disorder, preferably said aerosols comprise a pharmaceutical composition for the treatment of said pulmonary disease or disorder.

Also disclosed is a method of delivering aerosols to a subject in need thereof, the method comprising: providing an aerosol delivery device functionally associated with a processing circuitry having a yawn detector, wherein said aerosol delivery device comprises a controllable aerosol release mechanism; and actuating the controllable aerosol release mechanism, upon the processing circuity receiving indication of a yawn from the yawn detector, thereby releasing aerosols from the aerosol delivery device.

In addition to the exemplary aspects and embodiments described above, further aspects and embodiments will become apparent by reference to the figures and by study of the following detailed descriptions.

### BRIEF DESCRIPTION OF THE DRAWINGS

Examples illustrative of embodiments are described below with reference to figures attached hereto. In the figures, identical structures, elements or parts that appear in more than one figure are generally labeled with a same numeral in all the figures in which they appear. Alternatively, elements or parts that appear in more than one figure may be labeled with different numerals in the different figures in which they appear. Dimensions of components and features shown in the figures are generally chosen for convenience and clarity of presentation and are not necessarily shown in scale. The figures are listed below.
**Fig. 1** schematically illustrates a functional block diagram of a system for aerosols delivery, according to some embodiments;
**Fig. 2** schematically illustrates a system for aerosols delivery, according to some embodiments;
**Fig. 3** schematically illustrates a system for aerosols delivery, according to some embodiments;
**Fig. 4** schematically illustrates a device for aerosols delivery, according to some embodiments.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

In some embodiments there are provided herein systems for aerosols delivery. The system comprises an aerosol delivery device, such as, but not limited to a nebulizer or an inhaler; and a processing circuity configured to identify a yawn and to trigger a release of aerosols from said device upon identification of a yawn in a subject.

The combination of an aerosol delivery device with a processing circuity configured to identify a yawn allows optimal timing of an aerosol release to a subject in need thereof. In particular, this combination allows release of aerosol in the midst of a deep inhalation, which occurs during a yawn. Scheduling an aerosol delivery at the stage of deep inhalation improves the efficiency of delivering aerosol to the subject lungs.

In some embodiments there is provided a system for aerosols delivery, the system comprising an aerosol delivery device comprising a controllable aerosol release mechanism configured to release aerosols based on a control signal; a yawn detector configured to provide a yawn indicative signal in a subject; and a processing circuity configured to identify a yawn based on said yawn indicative signal and to provide a control signal to said aerosol release mechanism, thereby affect release of aerosols from said device.

The terms "aerosols" and "aerosol" as used herein are interchangeable and describe a nebulized solution or suspension consisting of very fine particles carried by a gas, which typically consists of air. The suspensions may be prepared from of a formulation in an inert liquid, such as water, wherein the formed dispersion usually comprises wet microspheres in air. Generally, aerosols include a gas-borne suspended phase, which is capable of being inhaled into the bronchioles or nasal passages. Aerosols may be produced, for example, by a metered dose inhaler or nebulizer, by a mist sprayer, or specifically by an aerosol delivery device according to the present invention. Typically, medical aerosols include dry powder compositions of pharmaceutical agent(s), employed in respiratory therapy for the treatment of medical conditions. Conditions susceptible to treatment with aerosols include, but are not limited to, bronchospasms, loss of compliance, mucosal edema, pulmonary infections and the like.

The term "nebulize" is used as a synonym for "transforming a liquid into an aerosol". Typically, nebulization occurs within a chamber where the aerosol is produced, by utilizing a source of energy, such as, a pneumatic or piezo-electric, which creates the aerosol.

In some embodiments, the aerosols is consisting of water. In some embodiments, the aerosols include a pharmaceutical composition. In some embodiments, the pharmaceutical composition is in a form of a dry powder.

A yawn is a reflex consisting of the simultaneous deep inhalation of air and the stretching of the eardrums, followed by an exhalation of breath. The average duration of a yawn is about six seconds, during which, the heart rate increases significantly.

Yawning most often occurs in adults immediately before and after sleep, during tedious activities and as a result of its contagious quality. It is commonly associated with tiredness, stress, sleepiness, or even boredom and hunger, though studies show it may be linked to the cooling of the brain. Yawns are often characterized by specific facial gestures, such as a wide opening of the mouth, stretching of the cheeks and eyebrows, opening or closing the eyelids, widening of the nostrils and wrinkling of the forehead. During yawning a person inhales deeply.

Due to the psychological effect of yawning, a yawn may be triggered by suggestive means. Yawning is often triggered by others yawning (e.g., seeing a person yawning, hearing the sound of yawning and even discussing yawning) and is a typical example of positive feedback. Moreover, it was found that yawning in rats is related to the sense of smell and can be triggered by exposing the animals to specific odors.

In some embodiments, the processing circuitry is configured to identify a yawn based on recognition of at least one facial gesture associated with yawning. In some embodiments, the identification of a yawn in a subject is based on recognition of at least two facial gestures associated with yawning. In some embodiments, the identification of a yawn in a subject is based on recognition of at least three facial gestures associated with yawning.

In some embodiments, the yawn indicative signal is provided based on recognition of at least one facial gesture associated with yawning. In some embodiments, the yawn indicative signal is provided based on recognition of at least two facial gestures associated with yawning.

In some embodiments, the identification of a yawn in a subject is based on recognition of at least one sound of the subject. In some embodiments, the yawn indicative signal is provided based recognition of at least one sound of the subject.

In some embodiments, the identification of a yawn in a subject is based on pneumatic pressure. In some embodiments, the yawn indicative signal is provided based on pneumatic pressure.

Identification of a pneumatic pressure as a signal corresponding to yawning and/or formation of a yawn includes, but is not limited to, the measurement of pressure at the mouth/mouth cavity. Typically, yawning is associated with a temporary decrease in pressure. Thus, identification of a reduced pressure or a negative change in pressure relates to the phenomenon of yawning.

In some embodiments, the identification of a yawn in a subject is based on recognition of a change in pneumatic pressure. In some embodiments, the yawn indicative signal is provided based on recognition of a change in pneumatic pressure.

In some embodiments, the change in pneumatic pressure comprises a decrease in pneumatic pressure.

In some embodiments, the identification of a yawn in a subject is based on recognition of a change in pneumatic flow. In some embodiments, the yawn indicative signal is provided based on recognition of a change in pneumatic flow.

In some embodiments, the change in pneumatic flow comprises an increase in pneumatic flow.

In some embodiments, the processing circuitry is configured to predict a yawn based on the yawn indicative signal, and to provide a control signal to the aerosol release mechanism, based on said prediction, thereby scheduling release of aerosols from the aerosol delivery device.

In some embodiments the prediction is based on recognition of at least one facial gesture associated with yawning. In some embodiments the prediction is based on recognition of at least two facial gestures associated with yawning.

In some embodiments the prediction is based on recognition of at least one sound of the subject.

In some embodiments the prediction is based on recognition a change in pneumatic pressure.

In some embodiments the prediction is based on recognition a change in pneumatic flow.

In some embodiments the yawn detector is configured to detect motion, sound, pneumatic flow, pneumatic pressure or any combination thereof. Each possibility represents a separate embodiment.

In some embodiments the yawn detector is configured to detect motion.

In some embodiments the yawn detector comprises a camera, a microphone, an air flow meter, a pressure gauge or any combination thereof.

In some embodiments the yawn detector comprises a camera.

In some embodiments the identification of the yawn is facilitated by a facial recognition program capable of recognizing facial gestures associated with yawning.

In some embodiments the facial recognition program is installed in the processing circuitry.

In some embodiments the processing circuitry comprises a mobile electronic device. In some embodiments the processing circuitry includes in a mobile electronic device.

In some embodiments the processing circuitry comprises a personal computer, a desktop computer, a laptop computer, a tablet, a phablet, smartwatch or a smartphone. Each possibility represents a separate embodiment. In some embodiments the processing circuitry comprises a tablet, a phablet a smartwatch or a smartphone.

In some embodiments the facial recognition program is a software or an application. In some embodiments the facial recognition program is embedded in the processing circuitry.

In some embodiments the facial recognition program includes a yawning detection algorithm.

In some embodiments the facial recognition program is configured to monitor the facial gestures.

In some embodiments the facial recognition program is configured to analyze data relating to the facial gestures associated with yawning.

In some embodiments the facial recognition program is further configured to decide if a yawn occurs. In some embodiments said facial recognition program is configured to decide when a yawn occurs. In some embodiments said facial recognition program is configured to predict when a yawn is expected to occur.

In some embodiments said facial recognition program is configured to decide if a yawn occurs based on at least one facial gesture associated with a yawn. In some embodiments said facial recognition program is configured to decide if a yawn occurs based on at least two facial gestures associated with a yawn.

In some embodiments said facial recognition program is configured to decide if a yawn occurs based on recognition of at least one sound of the subject.

In some embodiments said facial recognition program is configured to decide if a yawn occurs based on recognition a change in pneumatic pressure.

In some embodiments said facial recognition program is configured to decide if a yawn occurs based on recognition a change in pneumatic flow.

In some embodiments said facial recognition program is configured to decide when a yawn occurs based on at least one facial gesture associated with a yawn. In some embodiments said facial recognition program is configured to decide when a yawn occurs based on at least two facial gestures associated with a yawn.

In some embodiments said facial recognition program is configured to decide when a yawn occurs based on recognition of at least one sound of the subject.

In some embodiments said facial recognition program is configured to decide when a yawn occurs based on recognition a change in pneumatic pressure.

In some embodiments said facial recognition program is configured to decide when a yawn occurs based on recognition a change in pneumatic flow.

In some embodiments said facial recognition program is configured to predict when a yawn occurs based on at least one facial gesture associated with a yawn. In some embodiments said facial recognition program is configured to predict when a yawn occurs based on at least two facial gestures associated with a yawn.

In some embodiments said facial recognition program is configured to predict when a yawn occurs based on recognition of at least one sound of the subject.

In some embodiments said facial recognition program is configured to predict when a yawn occurs based on recognition a change in pneumatic pressure.

In some embodiments said facial recognition program is configured to predict when a yawn occurs based on recognition a change in pneumatic flow.

In some embodiments the facial gestures comprise a deep inhalation maneuver.

In some embodiments the facial gestures associated with a yawn comprise pre-yawning facial gestures.

In some embodiments the facial recognition program is further configured to provide a command to the processing circuitry to provide a control signal to the aerosol delivery device, thereby affect release of aerosols from the device.

In some embodiments the command is provided based on said decision obtained upon occurrence of a yawn. In some embodiments the command is provided based on said decision when a yawn occurs. In some embodiments the command is provided based on said prediction when a yawn occurs.

In some embodiments said command is given immediately upon the decision if or when a yawn occurs.

The term "immediately" as used herein refers to a time scale of fractions of a second or at most a few seconds, and no more than six seconds, which is the estimated duration of a yawn.

In some embodiments said command is given upon said prediction when a yawn occurs, such that the release of aerosols by the aerosol delivery device occurs during deep inhalation. In some embodiments said command is given upon said prediction when a yawn occurs, such that the release of aerosols by the aerosol delivery device occurs during the first second (namely, during 0 sec < tₐₑᵣₒₛₒₗ ≤ 1 sec, wherein tₐₑᵣₒₛₒₗ refers to the time of aerosol release) of yawning. In some embodiments said command is given upon said prediction when a yawn occurs, such that the release of aerosols by the aerosol delivery device occurs after the first second of yawning and before or during the consecutive second (namely, during 1 sec < tₐₑᵣₒₛₒₗ ≤ 2 sec). In some embodiments said command is given upon said prediction when a yawn occurs, such that the release of aerosols by the aerosol delivery device occurs during the third second of yawning (namely, during 2 sec < tₐₑᵣₒₛₒₗ ≤ 3 sec). In some embodiments said command is given upon said prediction when a yawn occurs, such that the release of aerosols by the aerosol delivery device occurs during the fourth second of yawning (namely, during 3 sec < tₐₑᵣₒₛₒₗ ≤ 4 sec). In some embodiments said command is given upon said prediction when a yawn occurs, such that the release of aerosols by the aerosol delivery device occurs during the fifth second of yawning (namely, during 4 sec < tₐₑᵣₒₛₒₗ ≤ 5 sec). In some embodiments said command is given upon said prediction when a yawn occurs, such that the release of aerosols by the aerosol delivery device occurs during the sixth second of yawning (namely, during 5 sec < tₐₑᵣₒₛₒₗ ≤ 6 sec).

In some embodiments said command is given upon said prediction when a yawn occurs, such that the release of aerosols by the aerosol delivery device occurs during the first two seconds of yawning (namely, during 0 sec < tₐₑᵣₒₛₒₗ ≤ 2 sec); the first three seconds of yawning (namely, during 0 sec < tₐₑᵣₒₛₒₗ ≤ 3 sec); the first four seconds of yawning (namely, during 0 sec < tₐₑᵣₒₛₒₗ ≤ 4 sec); the first five seconds of yawning (namely, during 0 sec < tₐₑᵣₒₛₒₗ ≤ 5 sec); or the first six seconds of yawning (namely, during 0 sec < tₐₑᵣₒₛₒₗ ≤ 6 sec).

In some embodiments said facial gestures include, but are not limited to, layout, positions, movements, shift, shapes, alterations, adjustments, arrangements, orientations, locations, contractions, expansions, spreading, stretching, enlargement, distortion, deviation, maneuvers, outline and/or appearance of at least one element of the face of a user. Each possibility represents a separate embodiment.

In some embodiments said elements include, but not are limited to, mouth, lips eye(s), jaw, ear(s), nose, nostrils, cheeks, eyebrow(s), neck facial skin and/ or forehead. Each possibility represents a separate embodiment.

In some embodiments the facial gestures associated with a yawn include any one or more of wide opening of the mouth, expansion of the nostrils and closing of the eyes.

In some embodiments the processing circuitry is functionally associated with a camera. In some embodiments the processing circuitry is connected to the camera by an electric cable. In some embodiments the processing circuitry is wirelessly associated with the camera. In some embodiments the camera is contained within the processing circuitry.

In some embodiments the facial gestures associated with a yawn are provided by the yawn detector. In some embodiments the facial gestures associated with a yawn are provided by the camera.

In some embodiments the processing circuitry comprises a non-transitory memory storage unit. In some embodiments the processing circuitry is configured to send and receive computer readable data to the non-transitory memory storage unit.

In some embodiments the computer readable data includes data specific to a user, in order to identify the subject and thus identify facial gestures thereof. In some embodiments the data specific to a user is derived from photos of the user. In some embodiments the photos of the user include photos of the user yawning. In some embodiments the photos of the user include photos of the user not yawning. In some embodiments the photos of the user include photos of the user yawning and photos of the user not yawning.

In some embodiments the photos of the user are provided by the yawn detector. In some embodiments the photos of the user are provided by the camera.

In some embodiments the data specific to a user is derived from sounds of the user. In some embodiments the sounds of the user include yawning sounds of the user.

In some embodiments the sounds of the user are provided by the yawn detector. In some embodiments the sounds of the user are provided by the microphone.

In some embodiments the processing circuitry is equipped to receive the data specific to a user. In some embodiments the data specific to a user is derived from photos of the user, provided by the detector. In some embodiments the data specific to a user is derived audible records of the user, provided by the detector.

In some embodiments the processing circuitry is equipped to receive data relating to the facial gestures at least once per second. In some embodiments the processing circuitry is equipped to receive data relating to the facial gestures at least twice per second. In some embodiments the processing circuitry is equipped to receive data relating to the facial gestures at least five times per second. In some embodiments the processing circuitry is equipped to receive data relating to the facial gestures at least 10 times per second. In some embodiments the processing circuitry is equipped to receive data relating to the facial gestures at least 25 times per second. In some embodiments the processing circuitry is equipped to receive data relating to the facial gestures at least 50 times per second. In some embodiments the processing circuitry is equipped to receive data relating to the facial gestures at least 100 times per second. In some embodiments the processing circuitry is equipped to receive data relating to the facial gestures at least 1,000 times per second.

In some embodiments the processing circuitry is further configured to stimulate yawning in the subject.

In some embodiments the system further comprises a yawn stimulator configured to stimulate yawning.

In some embodiments the processing circuitry is functionally associated with the yawn stimulator.

In some embodiments the processing circuitry is connected to the yawn stimulator by an electric cable. In some embodiments the processing circuitry is wirelessly associated with the yawn stimulator. In some embodiments the yawn stimulator is contained within the processing circuitry.

In some embodiments the yawn stimulator is configured to provide a still image, dynamic image, sound, scent, flavor, sensation, or any combination thereof. Each possibility represents a separate embodiment.

In some embodiments the yawn stimulator is configured to provide a still image, a dynamic image, sound or any combination thereof.

In some embodiments the yawn stimulator is configured to provide a still image.

In some embodiments the yawn stimulator is configured to provide a dynamic image.

In some embodiments the yawn stimulator is configured to provide sound(s).

In some embodiments the yawn stimulator is configured to provide a still image and sound(s).

In some embodiments the yawn stimulator is configured to provide a dynamic image and sound(s).

In some embodiments the yawn stimulator is configured to provide a change in temperature. In some embodiments the change in temperature comprises an increase in temperature.

In some embodiments the yawn stimulator comprises a display element.

In some embodiments the display element comprises a screen.

In some embodiments the yawn stimulator comprises an audio element.

In some embodiments the yawn stimulator comprises a display element and/or an audio element.

In some embodiments the audio element comprises at least one speaker.

In some embodiments the sound includes sounds of humans yawning, sounds of animals yawing, pronunciations and/or repetitions of words and/or sentences, monotonic sounds and/or stories and the like. Each possibility represents a separate embodiment.

In some embodiments the image includes a video, a figure or both. In some embodiments the image includes a video. In some embodiments the image includes a figure. In some embodiments the image includes a video and a figure. In some embodiments the figure includes a plurality of figures. In some embodiments the video includes a plurality of videos.

In some embodiments the video and/or the figures relate to yawning or weariness.

In some embodiments the video comprises at least one video of humans yawning and/or animals yawning.

In some embodiments the figure comprises at least one figure of humans yawning and/or animals yawning.

Without wishing to be bound by any theory or mechanism, yawning entails deep inhalation, which may improve drug delivery to the lungs, when using a nebulizer. Moreover, improvement of drug delivery to the lungs may lead to reduction of drug dosages, thus diminishing side effects. Yawning in humans is often triggered by sensing other yawning, and is a typical example of positive feedback. In other words, yawning may be contagious and subject to suggestibility.

In some embodiments the processing circuitry comprises a learning algorithm. In some embodiments the learning algorithm is configured to receive data relating to occurrences of said yawns in a subject and store the data in the non-transitory memory storage unit.

In some embodiments the data relating to occurrences of said yawns is derived from said still image, said dynamic image and/or said sound. In some embodiments the learning algorithm is configured to provide commands to the yawn stimulator based on said occurrences and said data, thereby enabling personalization of stimulation of yawning.

In some embodiments the yawning stimulation is activated manually. In some embodiments the yawning stimulation is manually activated by said subject or a caregiver.

In some embodiments the yawning stimulation is activated automatically. In some embodiments the yawning stimulation is activated automatically upon operation of the system. In some embodiments the yawning stimulation is activated automatically upon contact with the system. In some embodiments the yawning stimulation is activated automatically upon contact with the aerosol delivery device.

In some embodiments the aerosol delivery device is an inhaler or a nebulizer. In some embodiments the aerosol delivery device is an inhaler. In some embodiments the aerosol delivery device is a nebulizer.

In to some embodiments the aerosol delivery device comprises a container, configured to contain a liquid to be nebulized into said aerosols.

In some embodiments the aerosol delivery device comprises a nebulization chamber where the aerosols are produced.

In some embodiments the aerosol delivery device comprises a source of energy which creates the aerosol.

In some embodiments the source of energy comprises pneumatic energy or piezo-electric. In some embodiments the source of energy comprises pneumatic energy.

In to some embodiments the liquid comprises a pharmaceutical composition. In to some embodiments the aerosols comprise a pharmaceutical composition.

In some embodiments the pharmaceutical composition is for treating a pulmonary disease or disorder.

In some embodiments the pharmaceutical composition is selected from the group consisting of formoterol, albuterol, metaproterenol, terbutaline, bambuterol, clenbuterol, salmeterol, carmoterol, milveterol, indacaterol, saligenin- or indole- containing and adamantyl-derived β2 agonists, and pharmaceutically acceptable salts, esters, or isomers thereof. Each possibility represents a separate embodiment.

In some embodiments the pulmonary disease or disorder is selected the group consisting of asthma, inflammation, allergies, pulmonary vasoconstriction, allergic rhinitis, sinusitis, emphysema, impeded respiration, chronic obstructive pulmonary disease (COPD), pulmonary hypertension, bronchiectasis, respiratory distress syndrome parenchymatic and fibrotic lung diseases or disorders; cystic fibrosis, interstitial pulmonary fibrosis and sarcoidosis, tuberculosis and lung diseases and disorders secondary to HIV, pulmonary inflammation experienced with cystic fibrosis, and pulmonary obstruction experienced with cystic fibrosis. Each possibility represents a separate embodiment.

In some embodiments the aerosol delivery device is selected from the group consisting of: a pressurized meter dose inhaler, dry particle inhaler, soft mist inhaler, vibrating mesh nebulizer, jet nebulizer or ultrasonic wave nebulizer. Each possibility represents a separate embodiment of the present invention.

In some embodiments the aerosol delivery device is selected from the group consisting of: a pressurized meter dose inhaler, dry particle inhaler or soft mist inhaler. Each possibility represents a separate embodiment.

In some embodiments the aerosol release is a bolus aerosol release.

In some embodiments there is provided a system as described herein for use in the treatment of a pulmonary disease or disorder.

In some embodiments the aerosols comprise a pharmaceutical composition for the treatment of said pulmonary disease or disorder.

In some embodiments the pharmaceutical composition is selected from the group consisting of formoterol, albuterol, metaproterenol, terbutaline, bambuterol, clenbuterol, salmeterol, carmoterol, milveterol, indacaterol, saligenin- or indole- containing and adamantyl-derived β2 agonists, and pharmaceutically acceptable salts, esters, or isomers thereof. Each possibility represents a separate embodiment.

Also disclosed is a method of delivering aerosols to a subject in need thereof. The method comprising: providing an aerosol delivery device functionally associated with a processing circuitry having a yawn detector, wherein said aerosol delivery device comprises a controllable aerosol release mechanism; actuating the controllable aerosol release mechanism, upon the processing circuitry receiving indication of a yawn from the yawn detector, thereby releasing aerosols from the aerosol delivery device.

Actuation of the controllable aerosol release mechanism may be performed automatically upon the processing circuitry receiving indication of a yawn from the yawn detector.

The method may further comprise a step of receiving data relating to occurrences of said yawns in a subject by the processing circuitry. The method may further comprise a step of storing the data in the non-transitory memory storage unit.

The method may further comprise a step of gathering data relating to occurrences of said yawns in a subject.

Gathering data may comprise taking photos of the user by the detector. Gathering data may comprise recording sounds of the user by the detector.

The terms "subject" and "user" as used herein are interchangeable. The subject may be a human subject.

The method may further comprise a step of stimulating yawning in the subject.

The stimulation of yawning may be carried out by the processing circuitry. The stimulation of yawning may be carried out by a yawn stimulation program in the processing circuitry.

The method may be for the treatment of a respiratory disease or disorder.

The treatment of a respiratory disease or disorder may comprise alleviating shortness of breath.

The respiratory disease or disorder may be selected from the group consisting of asthma, inflammation, allergies, pulmonary vasoconstriction, allergic rhinitis, sinusitis, emphysema, impeded respiration, chronic obstructive pulmonary disease (COPD), pulmonary hypertension, bronchiectasis, respiratory distress syndrome parenchymatic and fibrotic lung diseases or disorders; cystic fibrosis, interstitial pulmonary fibrosis and sarcoidosis, tuberculosis and lung diseases and disorders secondary to HIV, pulmonary inflammation experienced with cystic fibrosis, and pulmonary obstruction experienced with cystic fibrosis.

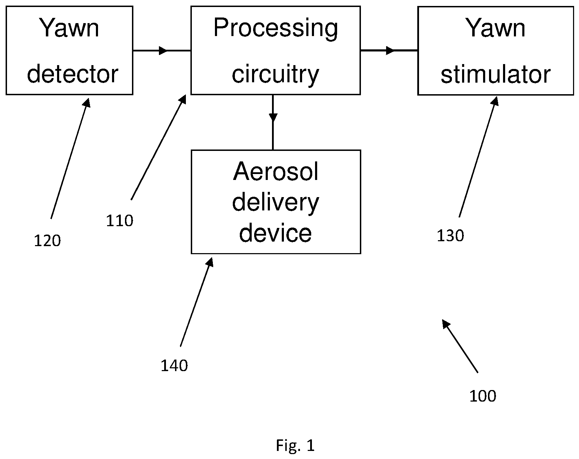

Reference is now made to **Fig. 1****,** which schematically shows a functional block diagram of a system **100** for aerosols delivery, according to some embodiments. System **100** comprises a processing circuitry **110,** which is functionally associated with a yawn detector **120,** a yawn stimulator **130** and an aerosol delivery device **140.**

Detector **120** is functionally associated with processing circuitry **110,** meaning that it may have wireless or wired connection to processing circuitry **110.** Yawn detector **120** is configured to receive and send electric signals to processing circuitry **110** through wired or wireless communication. Some of said electric signals are being referred to herein as yawn indicative signals.

In some embodiments yawn detector **120** is configured to detect motion, sound, pneumatic flow, change in pneumatic pressure, pneumatic pressure or any combination thereof. Appropriately, suitable detector may include video camera, still camera, microphone, EEG, microphone, motion detector, sound detector, air flow detector, air pressure detector and the like.

In some embodiments, system **100** may include a plurality of detectors, each functionally associated with processing circuitry **110,** wherein each one of said detectors is configured to detect a different physical attribute. For example, system **100** may include a motion detector, such as a camera, and a pneumatic flow detector, located on a mouthpiece of aerosol delivery device **140,** wherein both detectors are configured to receive and send electric signals to processing circuitry **110** through wired or wireless communication.

In some embodiments processing circuitry **110** comprises a computation unit, and may be an integral part of an external computer, such as a computation unit of a PC, laptop, smartphone, tablet and the like. In this case, yawn detector **120** may be an integral part of the mobile device, such as, but not limited to a camera and microphone of a smartphone.

In some embodiments processing circuitry **110** comprises a computation unit which is specifically designed for employment as a part of system **100.**

In case that processing circuitry **110** is an integral part of an external computer, such as a computation unit of a mobile device, the detector may also be an integral part of the mobile device, such a camera and microphone of a smartphone.

Processing circuitry **110** is functionally associated with yawn detector **120,** and is configured to receive a yawn indicative signal from it. Processing circuitry **110** is further configured to identify a yawn based on said yawn indicative signal. In some embodiments the identification of the yawn is facilitated by a recognition program installed in processing circuitry **110.** The program may be, for example, as a part of the hardware of processing circuitry **110** or added thereto as a software or application.

Processing circuitry **110** is further configured to predict a yawn based on said yawn indicative signal. In some embodiments the prediction of the yawn is facilitated by the recognition program.

The recognition program installed in processing circuitry **110** may include for example a facial recognition program, an audio recognition program, air pressure recognition program, air flow recognition program and the like. For example, the recognition program installed in processing circuitry **110** may include algorithms for analyzing one or more of a facial gesture signals, audio signals, air pressure signals and/ or air flow signals. In some embodiments, said algorithms are configured for providing output relating to predicting and/or determining when and/or if a yawn occurs, based on said analyzing. Appropriately, the yawn indicative signal may include any one or more of said facial gesture signals, audio signals, air pressure signals and/or air flow signals, such that the signals sent by yawn detector **120** to processing circuitry **110,** are subsequently being analyzed by the recognition program and influence said output of the algorithm. The recognition program may be further configured to send a command to processing circuitry **110** indicating it to send or schedule a control signal to a controllable aerosol release mechanism of aerosol delivery device **140.** The command may be based on said output of the algorithm.

In some embodiments, processing circuitry **110** is further configured predict a yawn based on the yawn indicative signal, and to provide a control signal to the aerosol release mechanism of aerosol delivery device **140,** based on said prediction, thereby scheduling release of aerosols from aerosol delivery device **140.** Similarly, processing circuitry **110** may be further configured to predict a yawn based said output of the algorithm, and to provide a control signal to the aerosol release mechanism of aerosol delivery device **140,** based on said prediction, thereby scheduling release of aerosols from aerosol delivery device **140.**

Processing circuitry **110** is also functionally associated with aerosol delivery device **140,** meaning that it may have wireless or wired connection to delivery device **140.** In particular, processing circuitry **110** may be functionally associated with a controllable aerosol release mechanism in aerosol delivery device **140,** and configured to send it control signal, thereby affecting release of aerosols from delivery device **140.** Processing circuitry **110** is further configured to schedule an aerosol release from aerosol delivery device **140** in a similar fashion.

In some embodiments processing circuitry **110** is equipped to receive the data specific to a user, comprising photos, videos and sounds of a user, provided by the detector. The processing circuitry **110** may incorporate this data in the recognition program, thereby modifying the algorithms for personalized yawn detection.

Processing circuitry **110** is further functionally associated with yawn stimulator **130,** meaning that they may include wireless or wired connection. Optionally, both yawn stimulator **130** and processing circuitry **110** are incorporated into a single device, such as, but not limited to, a mobile device. In another option, yawn stimulator **130** and processing circuitry **110** are incorporated into a single device specifically designed for employment as a part of system **100.**

Processing circuitry **110** provides data and commands to yawn stimulator **130** as to the stimulation of yawning. For example, processing circuitry **110** may include a non-transitory memory unit, where computer readable data is stored. Said computer readable data may correspond to yawn inducing elements, such as images, videos and sounds. Alternatively, said computer readable data may be stored in an external server, which is associated with processing circuitry **110** via wireless communication.

Yawn stimulator **130** is functionally associated with processing circuitry **110,** and is configured to stimulate yawning in the user. Yawn stimulator **130** comprises a display element, such as a screen, and an audio element, such as speakers, which are configured to provide still images, dynamic images and sounds. Yawn stimulator **130** may receive commands from processing circuitry **110,** relating to the stimulation of yawning in the user.

The sounds may include sound of humans yawning, sounds of animals yawing, pronunciations and/or repetitions of words and/or sentences, monotonic sounds and/or stories. Said words and/or sentences may be related to sleepiness and/or yawning, for example, the word 'yawn' and sentences indicating boredom weariness and desire to yawn. Said words and/or sentences and stories may be verbalized at a low pace thus further induce yawning.

The still images and dynamic images may include, but may not be limited to, video(s) and/ or figure(s) of humans yawning, video(s) and/ or figure(s) of animals yawing as well as video(s) and/ or figure(s) related to yawning or weariness.

Without wishing to be bound by any theory or mechanism, yawning entails deep inhalation, which may improve drug delivery to the lungs, when using a nebulizer. Moreover, improvement of drug delivery to the lungs may lead to reduction of drug dosages, thus diminishing possible related side effects. Yawning in humans is often triggered by sensing other yawning, and is a typical example of positive feedback. In other words, yawning may be contagious and subject to suggestibility.

Optionally, both yawn stimulator **130** and processing circuitry **110** are incorporated into a single device, such as, but not limited to, a mobile device. In this case, the display element may be a screen and the audio element may be a speaker(s), both incorporated into the mobile device, for example, the screen and speakers of a smartphone. In another option, yawn stimulator **130** and processing circuitry **110** are incorporated into a single device specifically designed for employment as a part of system **100.**

In some embodiments processing circuitry **110** further comprises a non-transitory memory storage unit and a learning algorithm. In this case, processing circuitry **110** is configured to receive data from yawn detector **120** relating to occurrences of yawns in a subject, and store the data in the non-transitory memory storage unit. Thereafter, the data is analyzed by the learning algorithm, and the learning algorithm may adjust the data and commands relating to yawn stimulation given by processing circuitry **110** to yawn stimulator **130,** thereby enabling personalization of yawn induction.

Aerosol delivery device **140** comprises a pharmaceutical composition, a mouthpiece and a controllable aerosol release mechanism. The controllable aerosol release mechanism is configured to receive control signals from processing circuitry **110,** thereby affecting the release of aerosols from aerosol delivery device **140.** Aerosol delivery device **140** further comprises a source of energy which creates the aerosol. In some embodiments the source of energy comprises pneumatic energy or piezo-electric energy. Said source of energy is mechanically activated by the controllable aerosol release mechanism, upon receiving the control signal from processing circuitry **110.** Immediately after forming, the aerosols are released through the mouthpiece.

The pharmaceutical composition is typically in the form of a solution, dispersion or suspension and is stored in a container, which may be refilled and/or replaced. The pharmaceutical composition is released as part of the aerosol, such that the release of aerosols from aerosol delivery device **140** entails release of the pharmaceutical composition. The pharmaceutical composition comprises a pharmaceutically active ingredients for the treatment of pulmonary disease or disorder, and its release is generally intended to provide a therapeutic effect over said disease or disorder, or their symptoms.

In the case when yawn detector **130** comprises an air flow detector or a pressure detector, it is preferable that these are placed over the mouthpiece of aerosol delivery device **140,** such that an accurate detection is made.

In some embodiments any one or more of yawn detector **130,** processing circuitry **110** and yawn detector **120** may be placed over, or integrated with, aerosol delivery device **140,** such that system **100** comprises a unified device.

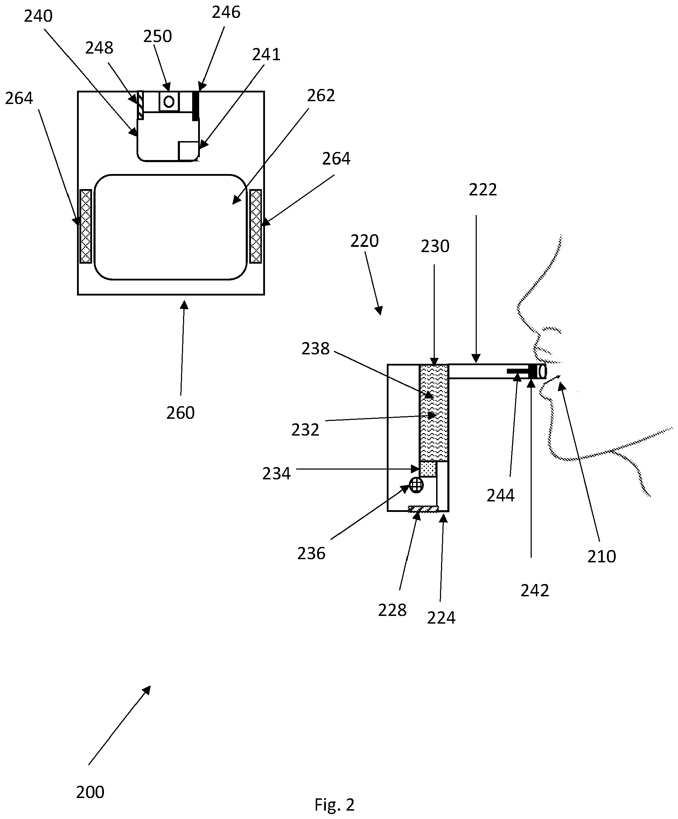

Reference is now made to **Fig. 2****,** which schematically illustrates a system for aerosol delivery **200** comprising a nebulizer **220,** wirelessly connected to a computation unit **240,** which is functionally associated with an air flow meter **242,** camera **250** and with a yawn stimulator **260** comprising a screen **262** and speakers **264.**

Camera **250** has wired connection to computation unit **240.** Camera **250** is configured to receive and send yawn indicative electric signals to computation unit **240.** Camera **250** is configured to detect motion and to acquire electronic motion pictures. Camera **250** is further configured to transform said electronic motion pictures to computer readable data and to send said data to computation unit **240.**

Air flow meter **242** is located at a mouthpiece **222** of nebulizer **220.** Air flow meter **242** comprises a transmitter **244,** which is configured to send electric signals to computation unit **240** through an antenna **246** of computation unit **240.** Air flow meter **242** is wirelessly connected to computation unit **240** and is configured to wirelessly send yawn indicative electric signals to computation unit **240** using transmitter **244.** Air flow meter **242** is configured to measure air flow and changes in air flow. Air flow meter **242** is further configured to transform said measurements to computer readable data and to send said data to computation unit **240.**

Transmitter **244** is located on air flow meter **242** and is configured to translate measurements relating to air flow to electric signals transferrable by wireless communication.

Computation unit **240** is specifically designed for employment as a part of system **200.** Computation unit **240** comprises antenna **246** and a transmitter **248.**

Computation unit **240** is functionally associated with camera **250** and with air flow meter **242,** and is configured to receive electronic signals from them. The electric signals of camera **250** are received through wired connection, whereas the electric signals of air flow meter **242** are received wirelessly through antenna **246.**

Said electronic signals comprise computer readable data relating to electronic motion pictures and computer readable data relating to measure air flow and changes in air flow. Computation unit **240** is further configured to identify a yawn based on said computer readable data.

Computation unit **240** comprises a recognition program installed therein, such that said identification of a yawn is facilitated by said program based on said computer readable data. The recognition program installed in computation unit **240** includes algorithms for analyzing facial gestures associated with yawning, air flow values associated with yawning and changes thereof, which are indicative for yawning. Said algorithm is configured for providing output relating to predicting and/or determining when and/or if a yawn occurs, based on said analyzing. The recognition program is further configured to send a command to computation unit **240** which is in turn indicating it to send or schedule a control signal to a controllable aerosol release mechanism **224** of nebulizer **220.** The command may be based on said output of the algorithm.

Computation unit **240** is further configured to predict a yawn based on the command, and to provide a control signal to the aerosol release mechanism **224** of nebulizer **220,** based on said prediction, thereby scheduling release of aerosols from nebulizer **220.**

Computation unit **240** comprises transmitter **248.** Transmitter **248** is located on computation unit **240** and is configured to send wireless control signals to controllable aerosol release mechanism **224** of nebulizer **220,** through an antenna **228** of controllable aerosol release mechanism **224.** Consequently, computation unit **240** is functionally associated with controllable aerosol release mechanism **224** of nebulizer **220,** and is configured to send thereto control signal(s), thereby affecting release of aerosols from nebulizer **220.** Similarly, computation unit **240** is further configured to schedule an aerosol release from nebulizer **220** using said wireless control signals from transmitter **248** to antenna **228.**

Computation unit **240** includes a non- transitory memory unit **241,** where computer readable data is stored. The computer readable data includes data corresponding to yawn inducing elements, such as images, videos and sounds that induce yawning.

Computation unit **240** is connected to yawn stimulator **260.** As can be seen in **Fig. 2****,** yawn stimulator **260** and computation unit **240** are incorporated into a single device specifically designed for employment as a part of system **200.** Computation unit **240** provides data and commands to yawn stimulator **260** as to the stimulation of yawn.

Antenna **246** is located on computation unit **240** and is configured to receive electronic signals from air flow meter **242** via transmitter **244.** It is further configured to translate said electric signals transferrable by wireless communication to computer readable data and to transfer said data to computation unit **240.**

Transmitter **248** is located on computation unit **240** and is configured to wirelessly transmit control signals to a controllable aerosol release mechanism **224** of nebulizer **220.**

Yawn stimulator **260** comprises screen **262** and speakers **264.** It is functionally associated with computation unit **240,** and is configured to stimulate yawning in the user. Screen **262** is configured to provide still images and dynamic images. Speakers **264** are configured to provide sounds. Yawn stimulator **260** may receive commands from computation unit **240,** relating to the stimulation of yawning in the user.

The sounds may include sound of humans yawning, sounds of animals yawing, pronunciations and/or repetitions of words and/or sentences, monotonic sounds and/or stories. Said words and/or sentences may be related to sleepiness and/or yawning, for example, the word 'yawn' and sentences indicating boredom weariness and desire to yawn. Said words and/or sentences and stories may be verbalized at a low pace thus further induce yawning.

The still images and dynamic images may include but not limited to, video(s) and/ or figure(s) humans yawning, video(s) and/ or figure(s) of animals yawing as well as video(s) and/ or figure(s) related to yawning or weariness.

Nebulizer **220** comprises a container **230** comprising a liquid **232,** a mouthpiece **222** and a controllable aerosol release mechanism **224.** Nebulizer **220** is configured to release aerosols upon receiving a control signal from computation unit **240** to controllable aerosol release mechanism **224.**

Controllable aerosol release mechanism **224** is configured to receive control signals from computation unit **240,** thereby affecting the release of aerosols from nebulizer **220.** Nebulizer **220** further comprises a pneumatic energy source **234,** which creates the aerosol. Said control signal are received by antenna **228.**

Antenna **228** is located on nebulizer **220** and is configured to receive electronic control signals from computation unit **240** via transmitter **248.**

Pneumatic energy source **234** is located on container **230** and is configured to exert pneumatic energy on liquid **232,** thereby nebulizing it and creating an aerosol. It is mechanically activated by controllable aerosol release mechanism **224,** upon receiving the control signal from computation unit **240.** Immediately after forming, the aerosols are released through mouthpiece **222.**

Mouthpiece **222** is located at one end of nebulizer **220** and is designed to fit into a mouth of a user **210.** Mouthpiece **222** is located in proximity to container **230,** such that when used, the nebulized aerosols are ejected through mouthpiece **222** into the mouth of the user **210.**

Container **230** is located inside nebulizer **220** and in close proximity to pneumatic energy source **234,** to mouthpiece **222** and to manual switch **236.** It comprises liquid **232,** which may be refilled or otherwise, entire container **230,** can be switched with a new, analogous container.

Liquid **232** is located inside container **230** and comprises a solution, dispersion or suspension comprising a pharmaceutical composition **238.** Pharmaceutical composition **238** is released as part of the aerosol, such that the release of aerosols from nebulizer **220** entails release of pharmaceutical composition **238.** Pharmaceutical composition **238** comprises a medicine for treatment of pulmonary disease or disorder, and its release is generally intended to induce a therapeutic effect over said disease or disorder, or over their symptoms.

Nebulizer **220** further comprises a manual switch **236,** located on the controllable aerosol release mechanism **224,** such that upon decision of a user, the user may press the switch, thereby actuate the pneumatic energy source **234** and affect a release of aerosols from container **230** into the user's mouth **210** through mouthpiece **222.**

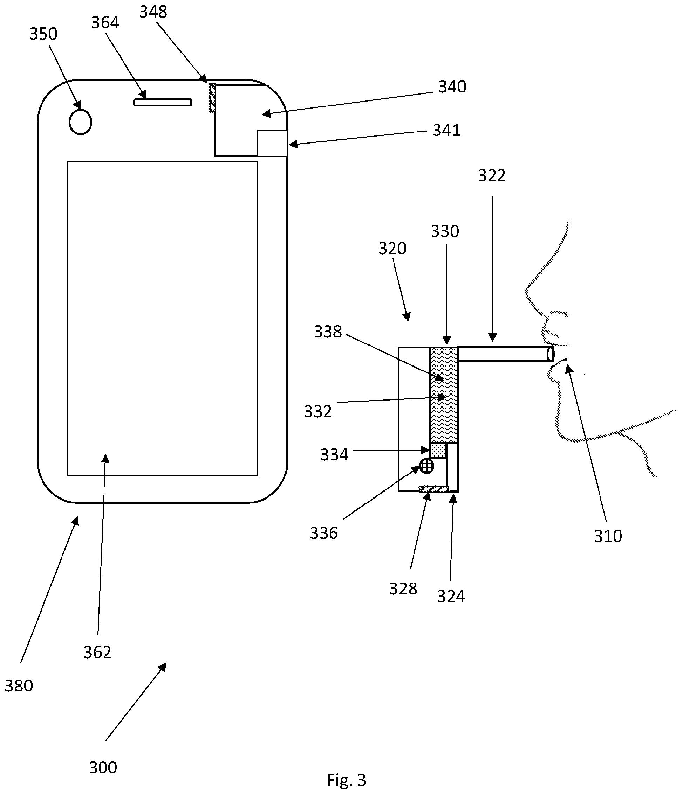

Reference is now made to **Fig. 3****,** which schematically illustrates a system for aerosol delivery **300** comprising a nebulizer **320,** wirelessly connected to a smartphone **380,** comprising a computation unit **340,** a camera **350,** a screen **362,** a speaker **364** and a transmitter **348.**

Smartphone **380** may be any type of commercial smartphone, rather than specifically designed for system **300,** as long as it includes a screen, speakers, camera and wireless communication through a transmitter, and as long as it includes an application or program as discussed hereinbelow.

Camera **350** is an integral part of smartphone **380.** Camera **350** has wired connection computation unit **340.** Camera **350** is configured to receive and send yawn indicative electric signals to computation unit **340.** Camera **350** is configured to detect motion and to acquire electronic motion pictures. Camera **350** is further configured to transform, through a wired connection, said electronic motion pictures to computer readable data and to send said data to computation unit **340.**

Computation unit **340** is an integral part of smartphone **380.** It is associated with transmitter **348** via an electric connection. Computation unit **340** is functionally associated with camera **350** and is configured to receive electronic signals from it through wired connection.

Said electronic signals comprise computer readable data relating to electronic motion pictures. Computation unit **340** is further configured to identify a yawn based on said computer readable data.

Computation unit **340** comprises a recognition program installed therein, such that said identification of a yawn is facilitated by said program based on said computer readable data. The recognition program comprises a smartphone application or a program. The recognition program installed in computation unit **340** includes algorithms for analyzing facial gestures associated with yawning. Said algorithm is configured for providing output relating to predicting and/or determining when and/or if a yawn is expected tooccur, based on said analyzing. The recognition program is further configured to send a command to computation unit **340** indicating it to send or schedule a control signal to a controllable aerosol release mechanism **324** of nebulizer **320.** The command is typically based on said output of the algorithm.

Computation unit **340** is further configured to predict a yawn based on the command, and to provide a control signal to the aerosol release mechanism **324** of nebulizer **320,** based on said prediction, thereby scheduling release of aerosols from nebulizer **320.**

Computation unit **340** is associated with transmitter **348** via an electric connection. Transmitter **348** is an integral part of smartphone **380** and is configured to send wireless control signals to controllable aerosol release mechanism **334** of nebulizer **320,** through an antenna **328** of controllable aerosol release mechanism **324.** Consequently, computation unit **340** is functionally associated with controllable aerosol release mechanism **324** of nebulizer **320,** and is configured to send it control signals, thereby affecting release of aerosols from nebulizer **320.** Similarly, computation unit **340** is further configured to schedule an aerosol release from nebulizer **320** using said wireless control signals from Transmitter **348** to antenna **328.**

Computation unit **340** includes a non- transitory memory unit **341,** where computer readable data is stored. The computer readable data includes data corresponding to yawn inducing elements, such as images, videos and sounds that induce yawning.

Computation unit **340,** screen **362** and speaker **364** are integral parts of smartphone **380** and so, computation unit **340** is connected to screen **362** and speaker **364** through wires. Computation unit **340** provides data and commands to screen **362** and speaker **364** as to the images and sound they produce.

Transmitter **348** is an integral part of smartphone **380** and is configured to wirelessly transmit control signals to a controllable aerosol release mechanism **324** of nebulizer **320.**

Smartphone **380** comprises screen **362** and speaker **364,** both of which are functionally associated with computation unit **340,** and are configured to stimulate yawning in the user. Screen **362** is configured to provide still images and dynamic images. Speaker **364** are configured to provide sounds. Both speaker **364** and screen **362** receive commands from computation unit **340,** relating to the stimulation of yawning in the user.

The sounds may include sound of humans yawning, sounds of animals yawing, pronunciations and/or repetitions of words and/or sentences, monotonic sounds and/or stories. Said words and/or sentences may be related to sleepiness and/or yawning, for example, the word 'yawn' and sentences indicating boredom weariness and desire to yawn. Said words and/or sentences and stories may be verbalized at a low pace thus further induce yawning.

The still images and dynamic images may include, but are not limited to, video(s) and/ or figure(s) of humans yawning, video(s) and/ or figure(s) of animals yawing as well as video(s) and/ or figure(s) related to yawning or weariness.

Nebulizer **320** comprises a container **330** comprising a liquid **332,** a mouthpiece **322** and a controllable aerosol release mechanism **324.** Nebulizer **320** is configured to release aerosols upon receiving a control signal from computation unit **340** to controllable aerosol release mechanism **324.**

Controllable aerosol release mechanism 324 is configured to receive control signals from computation unit **340,** thereby affecting the release of aerosols from nebulizer **320.** Nebulizer **320** further comprises a pneumatic energy source **334,** which creates the aerosol. Said control signals are received by antenna **328.**

Antenna **328** is located on nebulizer **320** and is configured to receive electronic control signals from computation unit **340** via transmitter **348.**

Pneumatic energy source **334** is located on container **330** and is configured to exert pneumatic energy on liquid **332,** thereby nebulizing it and create an aerosol. It is mechanically activated by controllable aerosol release mechanism **324,** upon receiving the control signal from computation unit **340.** Immediately after forming, the aerosols are released through mouthpiece **322.**

Mouthpiece **322** is located at one end of nebulizer **320** and is designed to fit into a mouth of a user **310.** Mouthpiece **322** is located in proximity to container **330,** such that when used, the nebulized aerosols are ejected through it into the mouth of the user **310.**

Container **330** is located inside nebulizer **320** and in close proximity to pneumatic energy source **334** to mouthpiece **322** and to a manual switch **336.** It comprises liquid **332,** which may be refilled or otherwise, entire container **330,** can be switched with a new, analogous container.

Liquid **332** is located inside container **330** and comprises a solution, dispersion or suspension comprising a pharmaceutical composition **338.** Pharmaceutical composition **338** is released as part of the aerosol, such that the release of aerosols from nebulizer **320** entails release of pharmaceutical composition **338.** Pharmaceutical composition **338** comprises a medicine for treatment of pulmonary disease or disorder, and its release is generally intended to have a therapeutic effect over said disease or disorder, or over its symptoms.

Nebulizer **320** further comprises a manual switch **336,** located on the controllable aerosol release mechanism **324,** such that upon decision of a user, the user may press the switch, thereby actuate the pneumatic energy source **334** and affect a release of aerosols from container **330** into his mouth **310** through mouthpiece **322.**

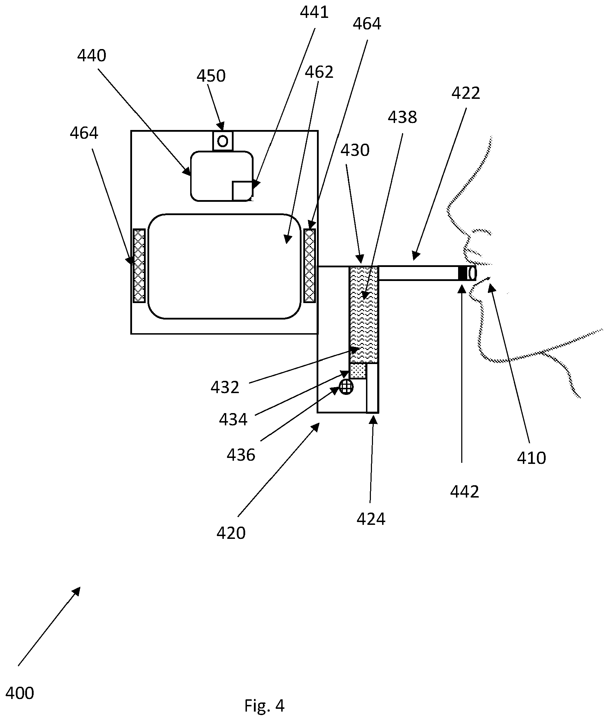

Reference is now made to **Fig. 4****,** which schematically illustrates a device for aerosol delivery **400** comprising a nebulizer **420,** a computation unit **440,** a camera **450,** an air flow meter **442,** a screen **462,** and speakers **464.**

As can be seen in **Fig. 4****,** each one of nebulizer **420,** computation unit **440,** camera **450,** air flow meter **442,** screen **462,** and speakers **464** is an integral component of device **400,** whereas each one of camera **450,** air flow meter **442,** screen **462,** and speakers **464** is connected through wired connection to computation unit **440.**

Camera **450** is an integral part of device **400.** It has wired connection to computation unit **440.** Camera **450** is configured to receive and send yawn indicative electric signals to computation unit **440.** Camera **450** is configured to detect motion and to acquire electronic motion pictures. Camera **450** is further configured to wirely transform said electronic motion pictures to computer readable data and to send said data to computation unit **440.**

Air flow meter **442** is located at a mouthpiece **422** of nebulizer **420.** Air flow meter **442** is configured to send wired electric signals, such as yawn indicative electric signals, to computation unit **440.** Air flow meter **442** is configured to measure air flow and changes in air flow. Air flow meter **442** is further configured to transform said measurements to computer readable data and to wirely send said data to computation unit **440.**

Computation unit **440** is an integral part of device **400.** It is functionally associated with camera **450** and is configured to receive electronic signals from it through wired connection. Computation unit **440** is functionally associated with camera **450** and with air flow meter **442,** and is configured to receive wired electronic signals from them.

Said electronic signals comprise computer readable data relating to electronic motion pictures and computer readable data relating to measure air flow and changes in air flow. Computation unit **440** is further configured identify a yawn based on said computer readable data.

Computation unit **440** comprises a recognition program installed therein, such that said identification of a yawn is facilitated by said program based on said computer readable data. The recognition program installed in computation unit **440** includes algorithms for analyzing facial gestures associated with a yawn, air flow values associated with a yawn and changes thereof, which are indicative for a yawn. The recognition program may be a part of an added software or a part of a hardware component of computation unit **440.**

The recognition program installed in computation unit **440** includes algorithms for analyzing facial gestures associated with a yawn. Said algorithm is configured for providing output relating to predicting and/or determining when and/or if a yawn occurs, based on said analyzing. The recognition program is further configured to send a command to computation unit **440** indicating it to send or schedule a control signal to a controllable aerosol release mechanism **424** of nebulizer **420.** The command is typically based on said output of the algorithm.

Computation unit **440** is further configured to predict a yawn based on the command, and to provide a control signal to the aerosol release mechanism **424** of nebulizer **420,** based on said prediction, thereby scheduling release of aerosols from nebulizer **420.**

Computation unit **440** is wirely associated with controllable aerosol release mechanism 424 of nebulizer **420,** and is configured to send it control signals, thereby affecting release of aerosols from nebulizer **420.** Similarly, computation unit **440** is further configured to schedule an aerosol release from nebulizer **420.**

Computation unit **440** includes a non- transitory memory unit **441,** where computer readable data is stored. The computer readable data includes data corresponding to yawn inducing elements, such as images, videos and sounds that induce yawning.

Computation unit **440,** screen **462** and speakers **464** are integral parts of device **400** and so, computation unit **440** is wirely connected to screen **462** and to speakers **464.** Computation unit **440** provides data and commands to screen **462** and speakers **464** as to the images and sound they produce.

Both screen **462** and speakers **464** are functionally associated with computation unit **440,** and are configured to stimulate yawning in the user. Screen **462** is configured to provide still images and dynamic images. Speakers **464** are configured to provide sounds. Both speakers **464** and screen **462** receive commands from computation unit **440,** relating to the stimulation of yawning in the user.

The sounds may include sound of humans yawning, sounds of animals yawing, pronunciations and/or repetitions of words and/or sentences, monotonic sounds and/or stories. Said words and/or sentences may be related to sleepiness and/or yawning, for example, the word 'yawn' and sentences indicating boredom weariness and desire to yawn. Said words and/or sentences and stories may be verbalized at a low pace thus further induce yawning.

The still images and dynamic images may include but not limited to, video(s) and/ or figure(s) of humans yawning, video(s) and/ or figure(s) of animals yawing as well as video(s) and/ or figure(s) related to yawning or weariness.

Nebulizer **420** comprises a container **430** comprising a liquid **432,** mouthpiece **422** and controllable aerosol release mechanism **424.** Nebulizer **420** is configured to release aerosols upon receiving a control signal from computation unit **440** to controllable aerosol release mechanism **424.**

Controllable aerosol release mechanism **424** is configured to receive control signals from computation unit **440,** thereby affecting the release of aerosols from nebulizer **420.** Nebulizer **420** further comprises a pneumatic energy source **434,** which creates the aerosol. Said control signals are received wirely from computation unit **440.**

Pneumatic energy source **434** is located on container **430** and is configured to exert pneumatic energy on liquid **432,** thereby nebulizing it and create an aerosol. It is mechanically activated by controllable aerosol release mechanism **424,** upon receiving the control signal from computation unit **440.** Immediately after forming, the aerosols are released through mouthpiece **422.**

Mouthpiece **422** is located at one end of nebulizer **420** and is designed to fit into a mouth of a user **410.** Mouthpiece **422** is located in proximity to container **430,** such that when used, the nebulized aerosols are ejected through it into the mouth of the user **410.**

Container **430** is located inside nebulizer **420** and in close proximity to pneumatic energy source **434,** to mouthpiece **422** and to a manual switch **436.** It comprises liquid **432,** which may be refilled or otherwise, entire container **430,** can be switched with a new, analogous container.

Liquid **432** is located inside container **430** and comprises a solution, dispersion or suspension comprising a pharmaceutical composition **438.** Pharmaceutical composition **438** is released as part of the aerosol, such that the release of aerosols from nebulizer **420** entails release of pharmaceutical composition **438.** Pharmaceutical composition **438** comprises a medicine for treatment of pulmonary disease or disorder, and its release is generally intended to have a mitigative effect over said disease or disorder, or over its symptoms.

Nebulizer **420** further comprises a manual switch **436,** located on the controllable aerosol release mechanism **424,** such that upon decision of a user, he may press the switch, thereby actuate the pneumatic energy source **434** and affect a release of aerosols from container **430** into his mouth **410** through mouthpiece **422.**

While a number of exemplary aspects and embodiments have been discussed above, those of skill in the art will recognize certain modifications, additions and sub-combinations thereof. It is therefore intended that the following appended claims and claims hereafter introduced be interpreted to include all such modifications, additions and sub-combinations as are within their true spirit and scope.

### EXAMPLES

### Example 1- Yawn-induced aerosol inhalation

A user in need for an aerosol delivery operates the operating mode by pressing the on switch, located on the nebulizer and selects setup mode by pressing the setup switch, located next to the on switch. This turns on a device having a screen, speakers, a camera and an internal computer having a facial recognition program, all of which are integral with the nebulizer. The screen, camera and the built-in speakers are simultaneously operated by the internal computer. At first, the screen displays a live dynamic video of the user, being taken by the camera. Simultaneously, a massage appears on the screen indicating to the user that he is in setup mode and that he should insert the nebulizer into his mouth and avoid yawning. The massage further indicates that the user is about to be photographed by the camera in a non-yawning position. The massage further indicates the time in which the photograph is about to be taken using a countdown indication. When the countdown reaches zero, a photographs of the user is being taken and a computer readable data corresponding to the photograph is temporarily saved in a non-transitory memory within the computer.

The screen displays the photograph, received from the computer and a second massage asking the user weather he agrees that the photograph serves as a reliable indication of how he looks while not yawning. The user can affirm or refuse that it serves as a reliable indication of how he looks while not yawning. The user affirms by pressing a second switch, located next to the on switch, and so, the photo data is permanently stored and the computer readable data corresponding to the photograph is encoded in the facial recognition program. If the user would refuse (by pressing a third switch, located next to the second switch), the photo data would be erased. Weather the user affirms or refuses the indication, the process (i.e. of preparing and taking a photo) is repeated until ten indicative photographs of the user in a non-yawning position are stored. The user is repeating the process until ten indicative photographs in a non-yawning position are stored.

After data of the user in a non-yawning position is gathered, a second stage of the setup commences. A third massage appears on the screen indicating to the user that he is about to be presented with a video clip and sound. The third massage further indicates that immediately when the user starts yawning, he should press the second switch. The third massage further indicates that immediately when the user finishes yawning, he should press the second switch again. A video clip is displayed, and the camera starts documenting the user's facial feature and elements, which were programmed beforehand. The elements include, the user's mouth, lips eyes, jaw, ears, nose, nostrils, chin, cheeks, eyebrows, neck, facial skin and forehead. Throughout the documentation, computer readable data corresponding to the documentation is temporarily stored in the non-transitory memory within the computer.

After a minute the user starts yawning and immediately presses the second switch. After few seconds the user finishes yawning and presses the second switch again.

The screen displays a part of the video, corresponding to the period between the first and second presses of the second switch, as derived from the stored computer readable data in the computer.

A fourth massage asking the user weather he agrees that the video serves as a reliable indication of how he looks while yawning. The user can affirm or refuse that it serves as a reliable indication of how he looks while yawning. The user affirms by pressing the second switch, and so computer readable data corresponding to a first video and a computer readable data corresponding to a second video are stored in the non-transitory memory within the computer. The first video includes a part of the video, corresponding to the period between three seconds before the first press of the second switch and the first press of the second switch. The first video is indicative of pre-yawning facial gestures of the user. The second video includes a part of the video, corresponding to the period between the first press of the second switch and the second press of the second switch. The second video is indicative of yawning facial gestures of the user.

Both videos are stored and encoded in the facial recognition program, which includes a yawn detection algorithm and a pre-yawn detection algorithm.

If the user would refuse (by pressing the third switch), the computer readable video data would be deleted from the non-transitory memory within the computer. Weather the user affirms or refuses the indication, the process (i.e. of showing a clip and taking a video) is repeated until ten indicative videos of the user in a yawning position are stored. The user is repeating the process until ten indicative videos in a yawning position are stored. A fifth massage, indicating the completion of setup is presented on the screen. The facial recognition program constructs a personalized yawn detection algorithm for the user.

After a while, the user charges the nebulizer with a solution containing a pharmaceutical composition of salbutamol inside a fitted package.

The user inserts the nebulizer into his mouth operates the operating mode by pressing the on switch. This turns on the screen, speakers, camera and internal computer. As a result of the operation, the screen and speaker display and sound figures, videos, clips and sounds of people and animals yawing. Throughout the showing of the clip, the camera monitors the facial gestures of the user thus gathering visual data relating to his facial gestures. The visual data gathered from the camera is continuously translated the facial recognition program within the internal computer.

The user watches and listens to the clip and the show begins to induce yawning in the user. After about a minute, the user naturally shows pre-yawning facial gestures (i.e. facial signs indicative that a yawn is about to take place). The camera, which continuously monitors the facial gestures of the user, transfers the corresponding computer readable data to the facial recognition program within the computer, which analyzes the computer readable data, using the pre-yawn detection algorithm. The program recognizes that a yawn is about to occur and schedules command to eject a bolus of the pharmaceutical composition in about four seconds.

Throughout the next four seconds the camera still monitors facial gestures of the user and transfers the corresponding data to the program. The program perform further computations in order to re-evaluate the propensity towards a user's yawn in the forthcoming seconds. This feature enables a false positive control to avoid ejections of the pharmaceutical composition in a non-yawning condition. Moreover, based on the continuously gathered computer readable data, the program modifies the timing of the command to eject a bolus of the pharmaceutical composition.

After few seconds a yawn commences and a deep inhalation of the user occurs. A command is given by the computer to the nebulizer to eject a bolus containing the solution of the pharmaceutical composition. A bolus of the solution is ejected as a spray and inhaled by the user.
After a few seconds, a sixth massage appears on the screen asking the user if the ejection was properly timed during a deep inhalation. The user affirms by pressing the second switch and the algorithms are modified accordingly. The system, including the screen, speakers, camera and internal computer, shuts down.

## Claims

1. A system (100, 200, 300) for aerosols delivery, the system (100, 200, 300) comprising:
an aerosol delivery device (140, 220, 320, 420) comprising a controllable aerosol release mechanism (224, 324, 424) configured to release aerosols based on a control signal;
**characterized in that** the system further comprises
a yawn detector (120, 242, 250, 350, 450, 442) configured to provide a yawn indicative signal in a subject; and
a processing circuity (110, 240, 340, 440) configured to identify a yawn based on said yawn indicative signal and to provide a control signal to said aerosol release mechanism (224, 324, 424), thereby affect release of aerosols from said device (140, 220, 320, 420),
preferably wherein said processing circuity (110, 240, 340, 440) is further configured to stimulate yawning in the subject.

2. The system (100, 200, 300) of claim 1, wherein identification of the yawn is facilitated by a facial recognition program capable of recognizing facial gestures associated with yawning,
preferably wherein said facial gestures comprise a deep inhalation maneuver.

3. The system (100, 200, 300) of claim 2, further comprising a yawn stimulator (130, 260) configured to stimulate said yawning.

4. The system (100, 200, 300) of claim 3, configured for said yawning stimulation being activated by said subject or a caregiver, or said yawning stimulation being activated automatically.

5. The system (100, 200, 300) of claim 1, wherein the aerosol delivery device (140, 220, 320, 420) is an inhaler or a nebulizer (220, 320, 420), or
wherein the aerosol delivery device (140, 220, 320, 420) is selected from the group consisting of: a pressurized meter dose inhaler, dry particle inhaler, or soft mist inhaler.

6. The system (100, 200, 300) of claim 1, wherein the processing circuitry (110, 240, 340, 440) is configured to predict a yawn based on the yawn indicative signal, and to provide a control signal to the aerosol release mechanism (224, 324, 424), based on said prediction, thereby schedule release of aerosols from the aerosol delivery device (140, 220, 320, 420).

7. The system (100, 200, 300) of claim 1, wherein said aerosol release is a bolus aerosol release, or
wherein said aerosol comprises a pharmaceutical composition.

8. The system (100, 200, 300) of claim 1 configured for use in the treatment of a pulmonary disease or disorder, preferably wherein said aerosols comprise a pharmaceutical composition for the treatment of said pulmonary disease or disorder.

9. The system (100, 200, 300) of claim 3, wherein said yawn stimulator (130, 260) is configured to provide still image, dynamic image, sound, scent, flavor, sensation or any combination thereof.

## Patentansprüche

1. System (100, 200, 300) zur Aerosolabgabe, wobei das System (100, 200, 300) folgendes umfasst:
eine Aerosolabgabevorrichtung (140, 220, 320, 420), die einen steuerbaren Aerosolfreisetzungsmechanismus (224, 324, 424) umfasst, der für eine Freisetzung von Aerosolen auf der Basis eines Steuersignals gestaltet ist;
**dadurch gekennzeichnet, dass** das System ferner folgendes umfasst:
einen Gähndetektor (120, 242, 250, 350, 450, 442), der so gestaltet ist, dass er ein Gähnen anzeigendes Signal in einem Subjekt bereitstellt;
eine Verarbeitungsschaltkreisanordnung (110, 240, 340, 440), die so gestaltet ist, dass sie ein Gähnen auf der Basis des ein Gähnen anzeigenden Signals identifiziert und ein Steuersignal an den Aerosolfreisetzungsmechanismus (224, 324, 424) bereitstellt, wodurch die Freisetzung von Aerosolen von der Vorrichtung (140, 220, 320, 420) bewirkt wird;
wobei die Verarbeitungsschaltkreisanordnung (110, 240, 340, 440) ferner so gestaltet ist, dass sie Gähnen in dem Subjekt stimuliert.

2. System (100, 200, 300) nach Anspruch 1, wobei die Identifizierung des Gähnens durch ein Gesichtserkennungsprogramm erleichtert wird, das mit Gähnen assoziierte Gesichtsgesten erkennen kann,
wobei die Gesichtsgesten vorzugsweise ein tiefes Einatmen umfassen.

3. System (100, 200, 300) nach Anspruch 2, das ferner einen Gähnstimulator (130, 260) umfasst, der zur Stimulation des Gähnens gestaltet ist.

4. System (100, 200, 300) nach Anspruch 3, das so gestaltet ist, dass es die Gähnstimulation durch das Subjekt oder einen Pfleger aktiviert, oder
wobei die Gähnstimulation automatisch aktiviert wird.

5. System (100, 200, 300) nach Anspruch 1, wobei die Aerosolabgabevorrichtung (140, 220, 320, 420) ein Inhalator oder ein Zerstäuber (220, 320, 420) ist), oder
wobei die Aerosolabgabevorrichtung (140, 220, 320, 420) ausgewählt ist aus der Gruppe bestehend aus: einem Druckdosierinhalator, einem Trockenpartikelinhalator oder einem Soft Mist-Inhalator.

6. System (100, 200, 300) nach Anspruch 1, wobei die Verarbeitungsschaltkreisanordnung (110, 240, 340, 440) so gestaltet ist, dass sie ein Gähnen auf der Basis des ein Gähnen anzeigenden Signals vorhersieht, und dass sie ein Steuersignal an den Aerosolfreisetzungsmechanismus (224, 324, 424) auf der Basis der Vorhersage bereitstellt, wodurch die Freisetzung von Aerosolen von der Aerosolabgabevorrichtung (140, 220, 320, 420) terminiert wird.

7. System (100, 200, 300) nach Anspruch 1, wobei die Aerosolfreisetzung eine Bolus-Aerosolfreisetzung ist, oder
wobei das Aerosol eine pharmazeutische Zusammensetzung umfasst.

8. System (100, 200, 300) nach Anspruch 1, das für eine Verwendung in der Behandlung einer Lungenerkrankung oder -störung gestaltet ist, wobei die Aerosole vorzugsweise eine pharmazeutische Zusammensetzung für die Behandlung der Lungenerkrankung oder -störung umfassen.

9. System (100, 200, 300) nach Anspruch 3, wobei der Gähnstimulator (130, 260) so gestaltet ist, dass er ein Standbild, ein dynamisches Bild, Ton, einen Duftstoff, einen Geschmacksstoff, eine Empfindung oder eine Kombination dieser bereitstellt.

## Revendications

1. Système (100, 200, 300) pour la distribution d'aérosols, le système (100, 200, 300) comprenant :
un dispositif de distribution d'aérosol (140, 220, 320, 420) comprenant un mécanisme de libération d'aérosol (224, 324, 424) pouvant être commandé conçu pour libérer des aérosols en fonction d'un signal de commande ;
**caractérisé en ce que** le système comprend en outre
un détecteur de bâillement (120, 242, 250, 350, 450, 442) conçu pour fournir un signal indicatif de bâillement chez un sujet ; et
un circuit de traitement (110, 240, 340, 440) conçu pour identifier un bâillement en fonction dudit signal indicatif de bâillement et pour fournir un signal de commande audit mécanisme de libération d'aérosol (224, 324, 424), affectant ainsi la libération des aérosols dudit dispositif (140, 220, 320, 420),
de préférence ledit circuit de traitement (110, 240, 340, 440) étant en outre conçu pour stimuler le bâillement chez le sujet.

2. Système (100, 200, 300) selon la revendication 1, l'identification du bâillement étant facilitée par un programme de reconnaissance faciale pouvant reconnaître les expressions faciales associées au bâillement,
de préférence lesdites expressions faciales comprenant une manœuvre d'inhalation profonde.

3. Système (100, 200, 300) selon la revendication 2, comprenant en outre un stimulateur de bâillement (130, 260) conçu pour stimuler lesdits bâillements.

4. Système (100, 200, 300) selon la revendication 3, conçu pour que ladite stimulation de bâillement soit activée par ledit sujet ou un soignant, ou que ladite stimulation de bâillement soit activée automatiquement.

5. Système (100, 200, 300) selon la revendication 1, le dispositif d'administration d'aérosol (140, 220, 320, 420) étant un inhalateur ou un nébuliseur (220, 320, 420), ou
le dispositif de distribution d'aérosol (140, 220, 320, 420) étant choisi dans le groupe constitué par : un inhalateur doseur à compteur pressurisé, un inhalateur à particules sèches ou un inhalateur à brouillard doux.

6. Système (100, 200, 300) selon la revendication 1, le circuit de traitement (110, 240, 340, 440) étant conçu pour prédire un bâillement en fonction du signal indicatif de bâillement, et pour fournir un signal de commande au mécanisme de libération d'aérosol (224, 324, 424), en fonction de ladite prédiction, programmant ainsi la libération d'aérosols à partir du dispositif de libération d'aérosol (140, 220, 320, 420).

7. Système (100, 200, 300) selon la revendication 1, ladite libération d'aérosol étant une libération d'aérosol en bolus, ou
ledit aérosol comprenant une composition pharmaceutique.

8. Système (100, 200, 300) selon la revendication 1 conçu pour être utilisé dans le traitement d'une maladie ou d'un trouble pulmonaire, de préférence ;
lesdits aérosols comprenant une composition pharmaceutique pour le traitement de ladite maladie ou dudit trouble pulmonaire.

9. Système (100, 200, 300) selon la revendication 3, ledit stimulateur de bâillement (130, 260) étant conçu pour fournir une image fixe, une image dynamique, un son, un parfum, une saveur, une sensation ou toute combinaison de ceux-ci.
